# Europäisches Patentamt

## European Patent Office

## Office européen des brevets

(19)

(11) Publication number: **0 012 534**
**B1**

(12)

# EUROPEAN PATENT SPECIFICATION

(45) Date of publication of patent specification: **27.01.82**

(21) Application number: **79302666.7**

(22) Date of filing: **22.11.79**

(51) Int. Cl.³: **C 07 C 1/04, B 01 J 29/06, B 01 J 23/74, B 01 J 37/00**

(54) Conversion of synthesis gas with iron-containing catalyst.

(30) Priority: **18.12.78 US 970300**

(43) Date of publication of application:
**25.06.80 Bulletin 80/13**

(45) Publication of the grant of the European patent:
**27.01.82 Bulletin 82/4**

(84) Designated Contracting States:
**DE GB SE**

(56) References cited:
**US - A - 4 086 262**
**US - A - 4 159 995**

(73) Proprietor: **MOBIL OIL CORPORATION**
**150 East 42nd Street**
**New York New York 10017 (US)**

(72) Inventor: **Butter, Stephen Allan**
**1150 Forge Road**
**Cherry Hill New Jersey 08034 (US)**
Inventor: **Schwartz, Albert B.**
**1901 John F. Kennedy Blvd.**
**Philadelphia Pennsylvania (US)**
Inventor: **Chester, Arthur Warren**
**517 Country Club Drive**
**Cherry Hill New Jersey 08003 (US)**

(74) Representative: **Cooper, John Anthony**
**Mobil Court 3 Clements Inn**
**London WC2A 2EB (GB)**

Courier Press, Leamington Spa, England.

# 0 012 534

## Conversion of synthesis gas with iron-containing catalyst

This invention is concerned with a method of preparing a synthesis gas (defined as mixtures of gaseous carbon oxides with hydrogen or hydrogen donors) conversion catalyst and to its use for the conversion of synthesis gas to naphtha.

Processes for the conversion of coal and other hydrocarbons, such as natural gas, to a gaseous mixture consisting essentially of hydrogen and carbon monoxide and/or dioxide are well known. Those of major importance depend either on the partial combustion of the fuel with an oxygen-containing gas, on the high temperature reaction of the fuel with steam, or on a combination of these two reactions. An excellent summary of the art of gas manufacture, including synthesis gas, from solid and liquid fuels is given in Encyclopedia of Chemical Technology, Edited by Kirk-Othmer, Second Edition, Volume 10, pages 353—433 (1966), Interscience Publishers, New York, New York.

It is also well known that synthesis gas will undergo conversion to reduction products of carbon monoxide, such as hydrocarbons, at from about 148.88°C (300°F) to about 454.44°C (850°F), under from about one to one thousand atmospheres pressure, over a fairly wide variety of catalysts. The Fischer-Tropsch process, for example, which has been most extensively studied, produces a range of liquid hydrocarbons, a portion of which have been used as low octane gasoline. Catalysts that have been studied for this and related processes include those based on iron, cobalt, nickel, ruthenium, thorium, rhodium and osmium, or their oxides.

Recently, it has been discovered that the conversion of synthesis gas into valuable products can be greatly enhanced by employing a special type of crystalline aluminosilicate zeolite exemplified by ZSM-5 in admixture with a carbon monoxide reduction catalyst. Thus, for example, United States Specification 4,086,262, discloses a process for the conversion of syngas by passing the same at elevated temperature over a catalyst which comprises an intimate mixture of a Fischer-Tropsch component and a special type of zeolite such as ZSM-5, and points out that the products produced are hydrocarbon mixtures which are useful in the manufacture of heating oil, high octane gasoline, aromatic compounds, and chemcial intermediates.

Although that Specification is primarily directed to multiple-particle composite catalysts, i.e., the crystalline aluminosilicate component (one particle) is physically admixed with the Fischer-Tropsch component (another particle), it's Example 5 does disclose a single particle iron-containing catalyst in an alumina matrix.

The patent and technical literature relating to the Fischer-Tropsch process is extensive and the various catalysts reported in the prior art have been used by themselves as well as in admixture with catalytically inactive supports such as kieselguhr. Although the reasons for using catalytically inactive supports have varied, it appears that one reason for using them was increased surface area of the Fischer-Tropsch component, another the control of the heat-requirements of the overall exothermic reactions.

It is also known in the art to admix a Fischer-Tropsch component with a material such as silica-alumina which is known to be catalytically active for the conversion of hydrocarbons.

According to the present invention a method of preparing a synthesis gas conversion catalyst comprises treating with carbon monoxide, at elevated temperature, a dried composite of iron oxide of particle size no greater than 200 microns, a hydrogel matrix and an acidic crystalline aluminosilicate zeolite having a silica to alumina ratio of at least 12 and a constraint index in the range 1 to 12. The composite may be prepared by forming a mixture of iron powder with the matrix and the zeolite and calcining such mixture in air, and the carbon monoxide used for the treating may itself be contained in synthesis gas. A preferred manner of drying the composite is spray-drying, which furnishes particles of a size suitable for fluidized bed use. The iron powder is typically of 10 to 90 microns particle size, and preferred siliceous matrices comprise silica, alumina or silica-alumina, while the preferred zeolite for use in the invention is zeolite ZSM-5, but similarly acting zeolites such as ZSM-11 and ZSM-12 are also favoured.

The conversion of synthesis gas to naphtha using the catalyst so prepared is carried out at a temperature of 500°F (260°C) to 600°F (315.55°C) and a pressure of 50 psig (446.07 kPa) to 1000 psig (6996.09 kPa). Fixed or fluidized bed may be employed.

The product naphtha has a boiling range of less than 204.44°C (400°F) at a 90% overhead which is defined as a $C_5+$ naphtha with an aromatic content of 5 to 25 weight percent wherein said fraction is at least 50 weight percent of the total hydrocarbons produced.

The matrix portion of the single particle fluid catalyst is not narrowly critical and suitable matrices include silica, alumina, silica-alumina, silica- zirconia, silica-magnesia, etc. One surprising feature of the novel process of this invention is that the catalysts which are employed may be unpromoted and yet they still exhibit high activity with little evident aging, and, in fact, are capable of converting syngas to the olefinic or aromatic naphtha product previously described while producing no more than 30 weight percent of methane plus ethane, based on total hydrocarbons. In fact, the use of promoters, which the prior art found necessary in previous iron-containing catalysts, is definitely not preferred due to the fact that most promoters are alkaline in nature and have a tendency to migrate to the acidic crystalline aluminosilicate zeolite component and to decrease the activity of the same. Therefore, it would appear that the single particle catalyst of the instant invention represents a significant departure from the teachings of the prior art in that not only are alkaline promoters not necessary for sustained operation but, in fact, are detrimental to the activity of the zeolitic component.

The catalyst can be prepared by adding the acidic crystalline aluminosilicate and either finely divided metallic iron or iron oxide to a hydrogel matrix before drying, homogenizing, and thereafter forming either fixed bed or fluid catalysts by conventional techniques, followed by calcination if metallic iron is used in order to convert it to iron oxide. The forming may however follow the addition of the iron or iron oxide to the hydrogel.

The amount of iron or iron oxide which is added is not narrowly critical and an amount sufficient to furnish 2.5 to 20 weight percent, preferably 2.5 to 10 weight percent, expressed as Fe, based on the finished catalyst, is usual.

Methods of making fluidized catalysts containing crystalline aluminosilicate zeolites and siliceous matrices are of course well known in the art. Thus, for example, a composite of a crystalline aluminosilicate zeolite and a siliceous matrix can be made by intimately mixing an aqueous alkali metal silicate with a particulate weighting agent, such as kaolin clay, desirably at room temperature. The mixture is then heated, generally to a temperature of from 37.77—71.11°C (100—160°F) and acid is added to adjust the pH of from about 8—10. The temperature is maintained for about 1—6 hours or longer. At this point, if a silica-zirconia matrix is desired, a zirconium salt is added, desirably as an aqueous solution thereof. Acid is then added to reduce the pH to about 4—7 and form a silica gel/weighting agent or a silica gel-zirconia gel/weighting agent slurry, which is then admixed with a slurry of an acidic crystalline aluminosilicate zeolite and the finely divided metallic iron or iron oxide. The resulting composite is then homogenized and treated with a source of ammonium ions or hydrogen ions in order to reduce the sodium content to a low level, desirably less than about 0.1% sodium, then spray dried to produce fluid size particles.

The most preferred weighting agent is kaolin clay, but other weighting agents may be substituted in whole or in part therefor. It is perfectly possible to omit the weighting agent altogether. The proportion of crystalline aluminosilicate zeolite to matrix is not narrowly critical and it can range from about 5—40 weight percent of the matrix.

When the drying is effected by spray-drying the temperature of the air (or other gas) entering the spray drier is ordinarily within the range of 260—537.77°C (500—1,000°F). The temperature used will depend on such factors as the quantity of material to be dried and the quantity of air used in the drying. The evaporization rate will vary depending on the quantity of air used in the drying. The temperature of the particles being dried is generally within the range of 65.55—148.88°C (150—300°F) at the completion of the drying, but preferably 93.33—176.66°C (200—350°F). The drying may be effected by a process in which the particles to be dried and a hot air stream are moving in the same direction for the entire drying period (concurrent drying) or where the hot stream flows in the opposite direction (counter-current drying), or by semi-concurrent drying, as is well known in the art.

If metallic iron is used to prepare the catalysts, it is necessary to calcine the same in air or oxygen-containing gas by heating at temperatures ranging from about 121.11°C (250°F) to about 593.33°C (1100°F) for periods of time ranging from about 1 to 24 hours or longer in order to convert the iron to iron oxide ($Fe_2O_3$). The catalyst must be treated, prior to its use for the conversion of syngas, with syngas or with CO. As opposed to prior art catalysts, the use of hydrogen alone has an adverse effect on catalytic properties and renders the catalyst totally unfit for use. Treatment with syngas or carbon monoxide is conveniently carried out at atmospheric pressure and at temperatures of about 287.77—343.33°C (550—650°F) for periods of time ranging from about 1/2 hour up to about 24 hours.

The iron powder or iron oxide powder used to prepare the catalysts should have a particle size of from 1 to 200 microns and should be relatively pure. Two forms of iron powder found to be particularly useful are electrolytically reduced iron (which was shown to contain irregularly shaped particles from about 1 to about 30 microns with a portion 100—200 microns in diameter) and submicron iron powder such as that supplied by Pyron Corporation having particles in the 40—90 micron range. The iron oxide used is preferably $Fe_2O_3$, although other forms of iron oxide can be used provided it is converted to $Fe_2O_3$ prior to the activation with carbon monoxide or syngas previously described. Powdered metallic iron is preferred over the use of iron oxide.

The acidic crystalline aluminosilicate component of the catalyst is characterized by a pore dimension greater than about 5 Angstroms, i.e. it is capable of sorbing normal paraffins, and it has a silica-to-alumina ratio of at least 12 and a constraint index within the range of 1 to 12. The significance and manner of determination of "constraint index" has been widely described in the patent literature, for instance in British Specification 1,446,522.

Constraint Index (CI) values for some typical zeolites including some not within the scope of this invention are:

| CAS | C.I. |
|---|---|
| Erionite | 38 |
| ZSM-5 | 8.3 |
| ZSM-11 | 8.7 |
| ZSM-35 | 6.0 |
| TMA Offretite | 3.7 |
| ZSM-38 | 2.0 |
| ZSM-12 | 2 |
| Beta | 0.6 |
| ZSM-4 | 0.5 |
| Acid Mordenite | 0.5 |
| REY | 0.4 |
| Amorphous silica-alumina | 0.6 |

A zeolite which when tested under any combination of conditions within the testing definition set forth in Specification 1,446,522 hereinabove referred to manifests a constraint index of 1 to 12 is intended to be included in the instant catalyst definition even if that same zeolite when tested under different conditions within that definition manifests a constraint index less than 1 or greater than 12.

Zeolites of particular interest are exemplified by ZSM-5, ZSM-11, ZSM-12, ZSM-35 and ZSM-38, identified by the x-ray diffraction data presented in U.S. Specifications 3,702,886, 3,709,886, 3,709,979, 3,832,449, 4,016,245 and 4,046,859 respectively.

The process of this invention is preferably carried out at temperatures ranging from 287.77°C (550°F) to about 304.44°C (580°F); at gas hourly space velocities (GHSV), ranging from 400 to 20,000 and more desirably from 500 to 6,000, based on fresh feed and total catalyst volume; at hydrogen to carbon oxides ratios from 0.5:1 to 2:1 and more preferably about 1:1; and pressures ranging from 446.07 kPa (50 psig) to 6996.09 kPa (1,000 psig) and more preferably from 1135.54 kPa (150 psig) to 2859.23 kPa (400 psig).

The following examples will illustrate the preparation of the catalyst and its use.

## Example 1

A silica-clay hydrogel was prepared by adding 153.2 cc of 97.1% sulfuric acid to a slurry of 698 grams of WP kaolin clay in 32.4 lbs of demineralized water and 2989 grams of Q-Brand sodium silicate at 48.88°C (120°F). After heating the mixture to 60°C (140°F) for two hours a solution containing 52.3 grams of aluminium sulfate in 209 cc of water was added, followed by 66.8 grams of sodium zirconium silicate in 648 cc water and the pH adjusted to 4.7 by adding sulfuric acid. After standing overnight the equivalent of 40% HZSM-5 and iron powder (electrolytically reduced iron of about 1—200 microns) in an amount sufficient to provide 5.8 weight percent iron based on total catalyst were added and the resulting gel was homogenized and $NH_4+$ exchanged, washed and dried.

## Example 2

The process of Example 1 was repeated with the exception that enough iron powder was added to provide a catalyst containing 17.1 weight percent iron.

## Example 3

The process of Example 1 was repeated with the exception that enough iron powder was added to provide a catalyst containing 14.8 weight percent iron.

## Example 4

The process of Example 1 was repeated with the exception that submicron iron powder of about 40—90 microns was used in an amount sufficient to obtain a catalyst having an iron content of 9.8 weight percent.

## Example 5

The process of Example 4 was repeated with the exception that sufficient iron powder was added to obtain a catalyst having an iron content of 16.7 weight percent.

### Example 6

Example 1 was repeated with the exception that submicron cobalt was used in place of the iron. Enough metallic cobalt was used to obtain a catalyst having a cobalt content of 5.0 weight percent.

### Example 7

The general procedure of Example 1 was repeated with the exception that the metallic iron was omitted during formation of the gel. The gel was dried and then impregnated with $Fe(NO_3)_3 \cdot 9H_2O$ in an amount sufficient to provide a composition containing 14.3 weight percent iron.

The various catalysts were then evaluated for the conversion of synthesis gas (1:1 $CO/H_2$) at 1480.28 kPa (200 psig), a WHSV of about 1 and at temperatures of about 301.66°C (575°F). All catalysts were air calcined at 537.77°C (1000°F) for 3 hours. However, comparison data is presented for some catalysts in both a calcined and uncalcined state. The data presented is that from the second day of use of the catalysts. The effect of preconditioning by treatment with either hydrogen or 1:1 mixtures of hydrogen and carbon monoxide at atmosphereic pressure for about 18 hours is also shown.

| Example | 1 | 1 | 2 |
|---|---|---|---|
| Run Time, Hours | 43 | 18 | 42 |
| WHSV | | | |
| CO Conversion, wt. % | 51.5 | <20 | 87.6 |
| $H_2$ Conversion, wt. % | 57.1 | | 67.1 |
| % wt. C Converted to: | | | |
|    Hydrocarbon | 64.8 | | 57.4 |
| Product Yield, wt. % | | | |
|    HC | 15.5 | | 24.9 |
|    $H_2O$ | 9.9 | | 6.8 |
|    $H_2$ | 3.0 | | 2.3 |
|    CO | 45.2 | | 11.6 |
|    $CO_2$ | 26.5 | | 54.4 |
| Hydrocarbon Composition, wt. % | | | |
|    $C_1$ | 17.3 | | 19.3 |
|    $C_2$ | 6.3 | | 9.6 |
|    $C_3$ | 5.0 | | 5.5 |
|    $C_4$ | 13.2 | Too Small to Analyze | 9.6 |
|    $C_5$ | 9.9 | | 8.8 |
|    $C_6+$ | 48.3 | | 47.3 |
| Olefins, wt. % by C No. | | | |
|    $C_2=$ | 30.2 | | 6.7 |
|    $C_3=$ | 29.0 | | 12.8 |
|    $C_4=$ | 19.9 | | 25.2 |
|    $C_5=$ | 18.4 | | 33.5 |
| $C_5$ Olefin Distribution, wt. % | | | |
|    $C_5=1$ | 2.2 | | 2.1 |
|    $2M1C_4=$ | 15.3 | | 16.7 |
|    $3M1C_4=$ | 0.7 | | 1.3 |
|    $T2C_5=$ | 12.4 | | 12.1 |
|    $C_2C_5=$ | 5.8 | | 5.9 |
|    $2M2C_4=$ | 63.5 | | 61.8 |
| $C_6+$ Aromatics, wt. % | 17.1 | | 8.2 |

| Example | 1 | 1 | 2 |
|---|---|---|---|
| Liq. Prod. 90% Pt., °C (D—2887) | 185—55 | | 185 |
| | (366°F) | | (365°F) |
| $C_5+$ | 58.2 | | 56.1 |
| Air Calcined | Yes | Yes | Yes |
| Pretreatment | A | B | A |

A = $CO + H_2$ at 315.55—323.88°C (600—615°F)

B = $H_2$ at 482.22°C (900°F)

| Example | 3 | 4 | 5 |
|---|---|---|---|
| Run Time, Hours | 18 | 18 | 18 |
| WHSV | | | |
| CO Conversion, wt. % | 69.4 | 61.4 | 93.0 |
| $H_2$ Conversion, wt. % | 62.9 | 54.9 | 68.6 |
| % wt. C Converted to: | | | |
| Hydrocarbon | 60.9 | 65.5 | 57.0 |
| Product Yield, wt. % | | | |
| HC | 20.6 | 18.6 | 25.0 |
| $H_2O$ | 8.9 | 11.3 | 8.1 |
| $H_2$ | 2.9 | 3.2 | 2.5 |
| CO | 28.2 | 35.8 | 6.5 |
| $CO_2$ | 39.8 | 31.0 | 58.0 |
| Hydrocarbon Composition, wt. % | | | |
| $C_1$ | 27.8 | 15.5 | 17.6 |
| $C_2$ | 8.0 | 5.0 | 6.4 |
| $C_3$ | 4.8 | 4.9 | 4.5 |
| $C_4$ | 9.6 | 12.3 | 9.1 |
| $C_5$ | 9.9 | 10.0 | 10.7 |
| $C_6+$ | 49.9 | 52.4 | 51.8 |
| Olefins, wt. % by C No. | | | |
| $C_2=$ | 15.7 | 19.7 | 10.0 |
| $C_3=$ | 16.1 | 0.4 | 19.4 |
| $C_4=$ | 30.4 | 13.1 | 26.3 |
| $C_5=$ | 43.2 | 24.1 | 35.8 |
| $C_5$ Olefin Distribution, wt. % | | | |
| $C_5=1$ | 2.2 | 2.0 | 21.0 |
| $2M1C_4=$ | 17.0 | 16.7 | 17.0 |
| $3M1C_4=$ | 1.4 | 1.1 | 1.4 |
| $T2C_5=$ | 12.8 | 11.9 | 12.2 |
| $C_2C_5=$ | 6.2 | 5.9 | 6.0 |
| $2M2C_4=$ | 60.5 | 62.4 | 61.3 |
| $C_6+$ Aromatics, wt. % | 11.7 | 19.8 | 14.4 |

| Example | 3 | 4 | 5 |
|---|---|---|---|
| Liq. Prod. 90% Pt., °C (D—2887) | 185 | 175 | 185.55 |
| | (365°F) | (347°F) | (366°F) |
| $C_5+$ | 59.6 | 53.6 | 64.9 |
| Air Calcined | Yes | Yes | Yes |
| Pretreatment | A | A | A |

A = CO + $H_2$ at 315.55—323.88°C (600—615°F)

B = $H_2$ at 482.22°C (900°F)

| Example | 5 | 6 | 6 |
|---|---|---|---|
| Run Time, Hours | | | |
| WHSV | | | |
| CO Conversion, wt. % | 0 | 0 | 0 |
| $H_2$ Conversion, wt. % | | | |
| % wt. C Converted to: | | | |
|     Hydrocarbon | | | |
| Product Yield, wt. % | | | |
|     HC | | | |
|     $H_2O$ | | | |
|     $H_2$ | | | |
|     CO | | | |
|     $CO_2$ | | | |
| Hydrocarbon Compositions, wt. % | | | |
|     $C_1$ | | | |
|     $C_2$ | | | |
|     $C_3$ | | | |
|     $C_4$ | | | |
|     $C_5$ | | | |
|     $C_6+$ | | | |
| Olefins, wt. % by C. No. | | | |
|     $C_2=$ | | | |
|     $C_3=$ | | | |
|     $C_4=$ | | | |
|     $C_5=$ | | | |
| $C_5$ Olefin Distribution, wt. % | | | |
|     $C_5=1$ | | | |
|     $2M1C_4=$ | | | |
|     $3M1C_4=$ | | | |
|     $T2C_5=$ | | | |
|     $C2C_5=$ | | | |
|     $2M2C_4=$ | | | |
| $C_6+$ Aromatics, wt. % | | | |

10

| Example | 5 | 6 | 6 |
|---|---|---|---|
| Liq.Prod. 90% Pt., °C. (D—2887) | | | |
| C$_5$ + | | | |
| Air Calcined | No | No | No |
| Pretreatment | A | B | A |

A = CO + H$_2$ at 315.55—323.88 °C (600—615°F)

B = H$_2$ at 482.22°C (900°F)

# 0 012 534

| Example | 6 | 7 |
|---|---|---|
| Run Time, Hours | | 18 |
| WHSV | | |
| CO Conversion, wt. % | 0 | 34.8 |
| $H_2$ Conversion, wt. % | | 46.4 |
| % wt. C Converted to: | | |
| Hydrocarbon | | 73.3 |
| Product Yield, wt. % | | |
| HC | | 11.9 |
| $H_2O$ | | 10.1 |
| $H_2$ | | 3.8 |
| CO | | 60.6 |
| $CO_2$ | | 13.6 |
| Hydrocarbon Composition, wt. % | | |
| $C_1$ | | 16.6 |
| $C_2$ | | 6.3 |
| $C_3$ | | 4.3 |
| $C_4$ | | 12.3 |
| $C_5$ | | 8.1 |
| $C_6+$ | | 52.5 |
| Olefins, wt. % by C No. | | |
| $C_2=$ | | 30.3 |
| $C_3=$ | | 9.8 |
| $C_4=$ | | 30.5 |
| $C_5=$ | | 35.9 |
| $C_5$ Olefin Distribution, wt. % | | |
| $C_5=1$ | | 2.0 |
| $2M1C_4=$ | | 16.4 |
| $3M1C_4=$ | | 1.1 |
| $T2C_5=$ | | 11.8 |
| $C2C_5=$ | | 5.7 |
| $2M2C_4=$ | | 62.9 |
| $C_6+$ Aromatics, wt. % | | 15.4 |

| Example | 6 | 7 |
|---|---|---|
| Liq. Prod. 90% Pt., °C (D—2887) | | 185.55 |
| | | (366 °F) |
| C$_5$+ | | 60.6 |
| Air Calcined | Yes | Yes |
| Pretreatment | A | A |

A = CO + H$_2$ at 315.55—323.88°C (600—615°F)

B = H$_2$ at 482.22°C (900°F)

From the above data, it can be seen that metallic cobalt was completely inoperative, irrespective of calcination or pretreatment (Example 6). It is also evident that calcination is absolutely necessary when employing metallic iron, from comparison of uncalcined Example 5 with Examples 1—4. The nature of pretreatment is also critical, as can be seen from comparison of the results obtained with the catalyst of Example 1 pretreated with and without carbon monoxide.

The Examples also demonstrate the superiority of the catalysts prepared from iron powder as opposed to conventional impregnation techniques. Examples 1 and 4 (iron powder) contained less iron than Example 7 (impregnation) and yet were more active for CO conversion. Example 3 which contained about the same amount of iron (14.8 weight percent) as Example 7 (14.3 weight percent) was almost twice as active.

Finally, the data demonstrate that the submicron iron i.e. 40—90 microns, was more active than the electrolytically reduced iron (1—200 microns). Example 2 had more iron than Example 5, i.e. 17.1 as opposed to 16.7 weight percent, but was not as active.

**Claims**

1. A method of preparing a synthesis gas conversion catalyst comprising treating with carbon monoxide, at elevated temperature, a dried composite of iron oxide of particle size no greater than 200 microns, a hydrogel matrix and an acidic crystalline aluminosilicate zeolite having a silica to alumina ratio of at least 12 and a constraint index in the range 1 to 12.

2. A method according to claim 1 wherein said composite is prepared by forming a mixture of iron powder with the matrix and the zeolite and calcining such mixture in air.

3. A method according to claim 1 or claim 2 wherein the carbon monoxide is contained in synthesis gas.

4. A method according to any of claims 1 to 3 wherein said composite is spray-dried.

5. A method according to any of claims 2 to 4 wherein the iron powder is of 10 to 90 microns particle size.

6. A method according to any preceding claim wherein the matrix comprises silica, alumina or silica-alumina.

7. A method according to any preceding claim wherein the zeolite is ZSM-5.

8. A synthesis gas conversion catalyst prepared by the method claimed in any of claims 1 to 7.

9. A process for converting synthesis gas to naphtha which comprises contacting the gas with the catalyst claimed in claim 8 at a temperature of 500°F (260°C) to 600°F (315.55°C) and a pressure of 50 psig (446.07 kPa) to 1000 psig (6996.09 kPa).

**Revendications**

1. Procédé de préparation d'un catalyseur de conversion du gaz de synthèse consistant à traiter, par le monoxyde de carbone à température élevée, un mélange séché d'oxyde de fer de dimension de particules non supérieure à 200 μ d'une matrice d'hydrogel et d'une zéolite d'aluminosilicate cristallisé acide ayant un rapport silice/alumine d'au moins 12 et un indice de contrainte dans la gamme de 1 à 12.

2. Procédé selon la revendication 1 dans lequel ledit mélange est préparé par formation d'un mélange de poudre de fer avec la matrice et la zéolite et calcination de ce mélange dans l'air.

3. Procédé selon la revendication 1 ou 2, caractérisé en ce que la monoxyde de carbone est contenu dans du gaz de synthèse.

# 0 012 534

4. Procédé selon l'une quelconque des revendications 1 à 3, dans lequel ledit mélange est séché par pulvérisation.

5. Procédé selon l'une quelconque des revendications 2 à 4, dans lequel la poudre de fer a une dimension de particules de 10 à 90 μ.

6. Procédé selon l'une quelconque des revendications précédentes, dans lequel la matrice renferme de la silice, de l'alumine ou de la silice-alumine.

7. Procédé selon l'une quelconque des revendications précédentes, dans lequel la zéolite est la ZSM-5.

8. Catalyseur de conversion de gaz de synthèse préparé par le procédé selon l'une quelconque des revendications 1 à 7.

9. Procédé pour convertir le gaz de synthèse en solvant naphta, qui comprend la mise en contact du gaz avec le catalyseur selon la revendication 8 à une température de 500°F (260°C) à 600°F (315,55°C) et sous une pression de 50 psig (446,07 kPa) à 1 000 psig (6 996,09 kPa).

## Patentansprüche

1. Verfahren zur Herstellung von Synthesegas-Umwandlungskatalysatoren, dadurch gekennzeichnet, daß mit Kohlenmonoxid bei erhöhter Temperatur ein getrocknetes Gemisch von Eisenoxid mit einer Teilchengröße von nicht mehr als 200 μm, einer Hydrogelmatrix und einem sauren kristallinen Aluminosilicat-Zeolithen mit einem Siliciumdioxid/Aluminiumoxid-Verhältnis von wenigstens 12 und einem Zwangsindex im Bereich von 1 bis 12 behandelt wird.

2. Verfahren nach Anspruch 1, dadurch gekennzeichnet, daß die Mischung durch Bildung eines Gemisches aus Eisenpulver mit der Matrix und dem Zeolithen gebildet wird und das entsprechende Gemisch in Luft calciniert wird.

3. Verfahren nach Anspruch 1 oder 2, dadurch gekennzeichnet, daß Kohlenmonoxid im Synthesegas enthalten ist.

4. Verfahren nach den Ansprüchen 1 bis 3, dadurch gekennzeichnet, daß die Mischung sprühgetrocknet wird.

5. Verfahren nach den Ansprüchen 2 bis 4, dadurch gekennzeichnet, daß das Eisenpulver eine Teilchengröße von 10 bis 90 μm hat.

6. Verfahren nach den vorhergehenden Ansprüchen, dadurch gekennzeichnet, daß die Matrix Siliciumdioxid, Aluminiumoxid oder Siliciumdioxid/Aluminiumoxid enthält.

7. Verfahren nach den vorhergehenden Ansprüchen, dadurch gekennzeichnet, daß der Zeolith aus ZSM-5 besteht.

8. Katalysator gemäß den Ansprüchen 1 bis 7 zur Verwendung für die Umwandlung von Synthesegas.

9. Verfahren zur Umwandlung von Synthesegas in Naphtha, dadurch gekennzeichnet, daß das Gas mit dem Katalysator gemäß Anspruch 8 bei einer Temperatur von 500°F (260°C) bis 600°F (315,55°C) und einem Druck von 50 psig (446,07 kPa) bis 1000 psig (6996,09 kPa) in Berührung gebracht wird.

14